# EUROPEAN PATENT APPLICATION

(11) **EP 1 906 105 A2**
(43) Date of publication of application: **02.04.2008**
(21) Application number: 07253604.8
(22) Date of filing: 11.09.2007
(51) Int. Cl.: F24F 3/16, F24H 3/04

(54) **A fan forced electric unit that incorporates a low power cold plasma generator and method of making the same**

(30) Priority: 13.09.2006 US 520009
(71) Applicant: Marley Engineered Products LLC, Bennettsville, SC 29512 (US)
(72) Inventor: Alexander, Joseph, Laurinburg 28352 North Carolina (US); King, Ronald, Bennettsville 29512 South Carolina (US); Sun, Zongli Julie, Minhang District Shanghai 200240 (CN)
(74) Representative: Maury, Richard Philip

(57) **Abstract**

An apparatus and method of using electrical unit capable of improving air qualities in an indoor area. The electrical unit having an air circulator configured to generate an air stream, a cold plasma generator operably connected to a first and a second high voltage wires, the cold plasma generator configured to produce a high voltage between the first and second high voltage wires, a first electrode electrically connected to the first high voltage wire; and a second electrode electrically connected to the second high voltage wire; the first and second electrodes configured to generate a high voltage electric field in the air stream.

## Description

### FIELD OF THE INVENTION

The present invention relates generally to indoor air quality improvement. More particularly, the present invention relates to a device for providing heat and improving the air quality of an indoor area using a cold plasma generator.

### BACKGROUND OF THE INVENTION

Air contamination is a serious health concern. It is a known concern that air can be contaminated by contaminants, such as, bacteria, viruses, allergens, and other airborne diseases. In some circumstances, these contaminants can cause us minor discomfort; however, in some serious cases, these contaminants can cause death. For example, during the 2003 SARS (Severe Acute Respiratory Syndrome) outbreak, 774 people died of this airborne virus.

In order to improve air quality, negative ion technologies have been employed. Some negative ion technology devices use oxygen atoms that have gained an electron. Negative oxygen ions purify the air by magnetically attracting to positively charged pollutants (for example: dust, pollen, smoke, and dander) until these newly-formed larger particles of pollutants and ions become too heavy to remain suspended in air. However negative oxygen ions have a very short life (2-6 minutes) and become inactive before they can circulate completely throughout a dwelling. Furthermore, a high-powered negative ion generator typically produces "black- wall effect," which is a hard-to-remove residue that settles on the wall or other surfaces near the ion generator. In addition, most negative ion generators use filters and require frequent maintenance.

Ozone generators are also used to purify air. Ozone generators generate ozone, which are activated oxygen containing three atoms of oxygen rather than the two atoms that we normally breath. Ozone has powerful bacteria killing effect. However, ozone is also known to be harmful to humans. Furthermore, the cost associated with ozone generators are generally high.

Accordingly, it is desirable to provide an electric unit that can eliminate pollutants and airborne viruses that is cost effective. Furthermore, it is desirable to provide an electric unit that does not cause 'black-wall effect." In addition, it is desirable to provide an electric unit that is not harmful to the user.

### SUMMARY OF THE INVENTION

The foregoing needs are met, to a great extent, by the present invention, wherein in one aspect an apparatus is provided that in some embodiments includes a fan forced electrical unit that incorporates a cold plasma generator to provide rapidly rising, short lasting electric pulse to purify air. In another embodiments a cold plasma generator is combined with other devices that process air uses as a heater.

In accordance with one embodiment of the present invention, an electrical unit is provided. The electrical unit includes an air circulator configured to generate an air stream; a cold plasma generator operably connected to a first and a second high voltage wires, the cold plasma generator configured to produce a pulse voltage differential between the first and second high voltage wires; a first electrode electrically connected to the first high voltage wire; and a second electrode electrically connected to the second high voltage wire; the first and second electrodes configured to generate a high pulse voltage electric field in the air stream.

In accordance with another embodiment of the present invention, an electrical apparatus is provided. The electrical apparatus includes means for circulating an air stream; means for generating high voltage connected to the means for circulating, means for generating high voltage operably connected to a first means and a second means for transmitting high voltage, the means for generating high voltage configured to produce a pulse voltage between the first means and the second means for transmitting high voltage; a first means for generating a high voltage electric field in the air stream electrically connected to the first means for transmitting high voltage; and a second means for generating a high voltage electric field in the air stream electrically connected to the second means for transmitting high voltage.

In accordance with yet another embodiment of the present invention, a method of making an electrical apparatus is provided. The method includes placing a cold plasma generator inside a housing; connecting a first and a second high voltage wires to the cold plasma generator; connecting a first electrode to the first high voltage wire; connecting a second electrode to the second high voltage wire; and placing an air circulator inside the housing, such that the air circulator moves air through an electric field generated by the first and second electrodes. The method further includes connecting a motor to the air circulator; placing a heating element inside the housing; connecting a thermo sensor to the heating element; connecting a thermal fuse between the heating element and the thermo sensor; and placing an indicator light in the housing.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1 is a partial exploded view of an electric heater according to a preferred embodiment of the invention.

FIG. 2 is a front view of an electric unit according to the present invention without the front cover and fan deck.

FIG. 3 is a cross-sectional view taken along the 3--3 in FIG. 2.

FIG. 4 is an exploded view of the fan-panel assembly according to the present invention showing various sub-parts.

FIG. 5 illustrates a first wiring diagram of an embodiment of the present invention.

FIG. 6 illustrates a second wiring diagram of another embodiment of the present invention.

### DETAILED DESCRIPTION

The invention will now be described with reference to the drawing figures, in which like reference numerals refer to like parts throughout. An embodiment in accordance with the present invention provides a fan forced electrical unit that incorporates a low power cold plasma generator to provide rapidly rising, short lasting electric pulse to purify air. The low power cold plasma generator can generate an electric pulse of about 3000 volts on the order of milliseconds.

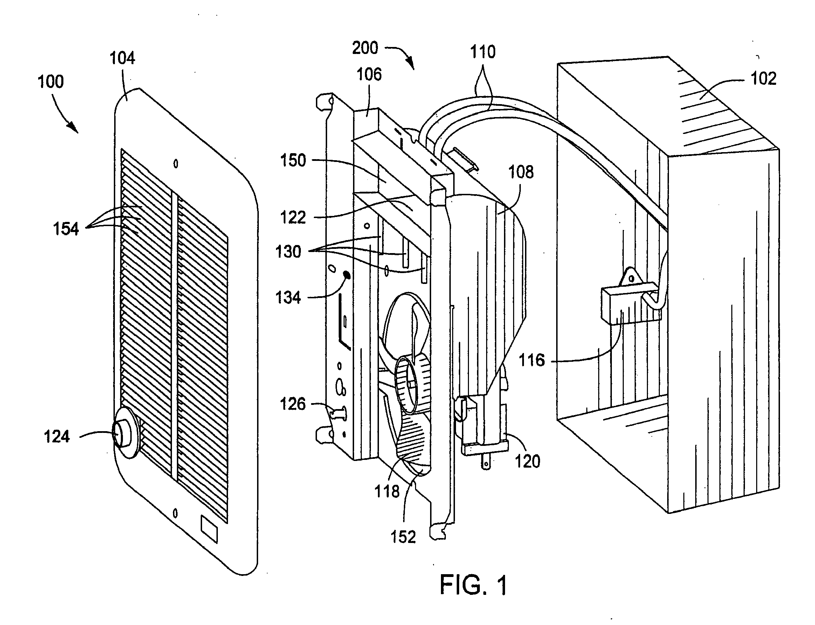

An embodiment of the present inventive apparatus is illustrated in FIG. 1. The electric unit 100 includes a back cover 102, a front cover 104, and a. fan-panel assembly 200.

The front cover 104 includes a knob 124. The front cover 104 has slits 154 to permit air to flow through the front cover 104. The front cover 104 is fitted over the back cover 102. In some embodiments of the invention the electric unit 100 is a portable space heater. The knob 124 is connected to the thermostat 126. A user may turn on the electric unit 100 by turning the knob 124 and may also adjust heat output of the electrical unit 100 using the knob 124. For example, the user can adjust the temperature by turning the knob clockwise or counter-clockwise.

The thermostat 126 is secured on the fan deck 106 and is electrically connected a heating element 122. The thermostat is configured to send a signal to the heating element 122 to turn on or off when the temperature raises above or descends below a predetermined temperature.

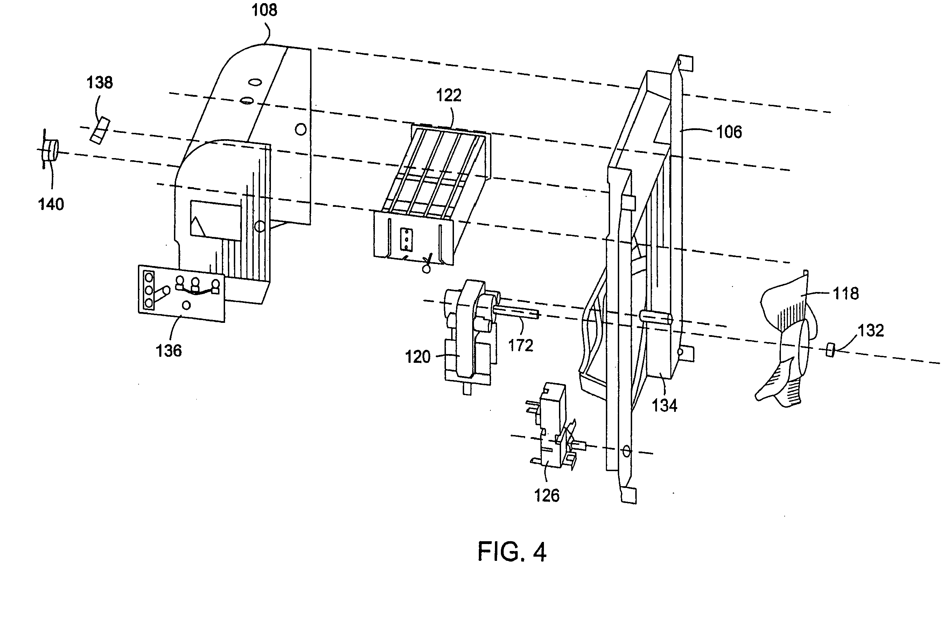

An indicator light 134 is also mounted on the fan-panel assembly 200. The indicator light 134 is electrically connected to the heating element 122, such that the indicator light 134 illuminates when either a thermo sensor 140 or the thermal fuse 138 fails (see FIG. 4).

The fan-panel assembly 200 has an opening 150 to facilitate air flow. The fan-panel assembly 200 has a fan opening 152 to allow a fan 118 to draw air into the electric unit 100 through the slits 154 in the front cover. The fan-panel assembly 200 includes slots 130 for mounting the heating element to a fan deck 106. An element mount 108 has a gradually curved rear section, and is secured on the backside of the fan deck 106, such that a heating element 122 is mounted between the fan deck 106 and the element mount 108. Furthermore, the heating element 122 is mounted below the opening 150, such that cold air passes through the heating element 122 and becomes warm, then exits the opening 150. A motor 120 is secured on the fan deck 106 and is operatively connected to the fan 118.

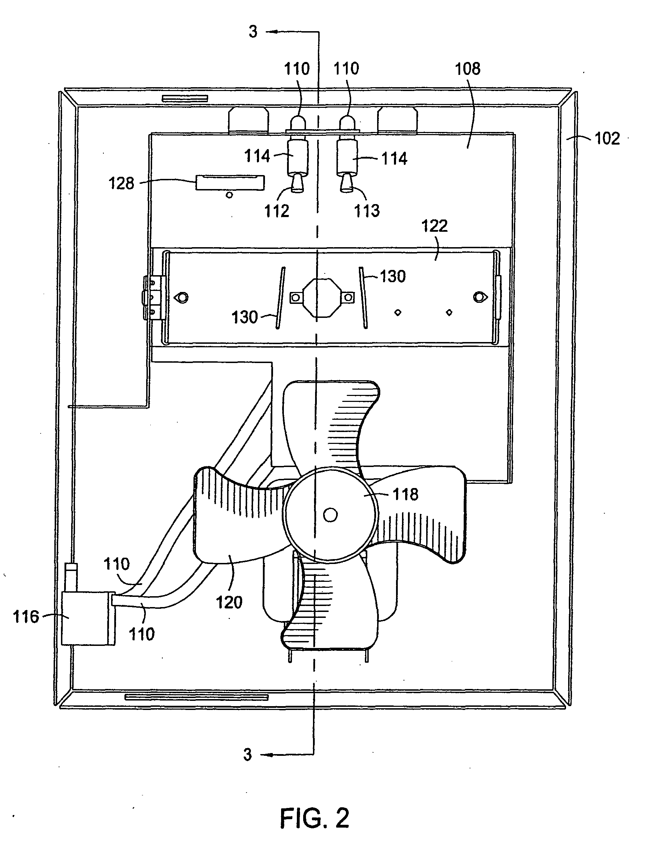

A cold plasma generator 116, also known as a plasma generator, is mounted on the back cover 102. Two high voltage wires 110 each having two ends are electrically connected to the cold plasma generator 116 at one of their ends and the other ends are connected to a first and a second electrodes 112, 113 as shown in FIG. 2. The two electrodes 112, 113 are oppositely charged and are at least in part covered by plastic brackets 114. The first and the second electrodes 112, 113 can be about 1.20 inches to about 1.30 inches apart. In some embodiments of the invention, the first and the second electrodes 112, 113 are about 1.26 inches apart. In some embodiments of the invention, the first and the second electrodes 112, 113 are carbon brushes.

The cold plasma generator 116 generates rapidly rising pulse of discharge between the first and the second electrodes 112, 113. The cold plasma generator may generate a pulse between about 2500 to about 6000 volts between the first and the second electrodes 112, 113. In some embodiments of the invention, the cold plasma generator 116 generates a pulse of about 3000 volts between the first and the second electrodes 112, 113. In some embodiments of the invention, the rapidly rising pulse has a cycle time on the order of a millisecond. In some embodiments of the invention; the rapidly rising pulse has a cycle of between about 1 to about 6 milliseconds. In some embodiments of the invention, the rapidly rising pulse has a cycle of between about 3 to about 4 milliseconds.

A rapid discharge is a process by which a pulse current, perhaps sustained, develops from an electrode with a high potential in a neutral fluid, usually air, by ionizing that fluid so as to create a plasma around the electrode. The ions generated eventually are passed to nearby area by air stream, or recombine to form neutral gas molecules. Thus, the cold plasma generator 116 does not produce harmful by products in the process. Therefore, it is more suitable than negative ion technologies or ozone generators for purifying air in an indoor area.

The electrodes 112, 113 are mounted on the element mount 108 and above the opening 150 of the fan panel 106, such that when warm air exits the opening 150 and passes through the electrodes 112, 113, the cold plasma generator 116 will generate a high energy pulse and split water molecules suspended in the air into oppositely charged hydrogen and hydroxyl ions. The negative hydroxyl ions will bond will bacteria or viruses in the air, interrupting their metabolism and preventing them from further reproduction.

The electric unit 100 does not create "black-wall effect." "Black-wall effect" is created by negative charged particles, This effect is used in dust collection. The electric unit 100 creates plasma, positively charged hydrogen ion and negatively charged hydroxyl ion, which has little effect to dust. Thus, unlike negative ion generator, the electric unit 100 will not creat "black-wall effect."

FIG. 2 is a front view of the electric unit 100 according to an embodiment of the present invention with the front cover 104 and the fan deck 106 removed. The fan deck 106 can be made of metal with the openings 150,152 (see FIG 1). The element mount 108 is secured behind the fan deck 106 and the heating element 122 is mounted in between the element mount 108 and the fan deck 106. The heating element 122 is secured on the fan deck 106 and the element mount 108 through slots 130.

As discussed above, the cold plasma generator 116 is connected to two high voltage wires 110 and which are connected to electrodes 112, 113. The electrodes 112, 113 are carbon brushes and are in part covered by plastic brackets 114 for electrical insulation. Therefore, the plastic brackets 114 will not effect the discharge process. Furthermore, the plastic brackets 114 can be configured to secure the electrodes 112, 113 on the fan deck 106.

The element mount 108 has an opening 128 for adapting a thermal fuse 128. The thermal fuse 128 is electrically connected between the heating element and the thermo sensor. The thermal fuse 128 is a fail-safe device for the electric unit 100.

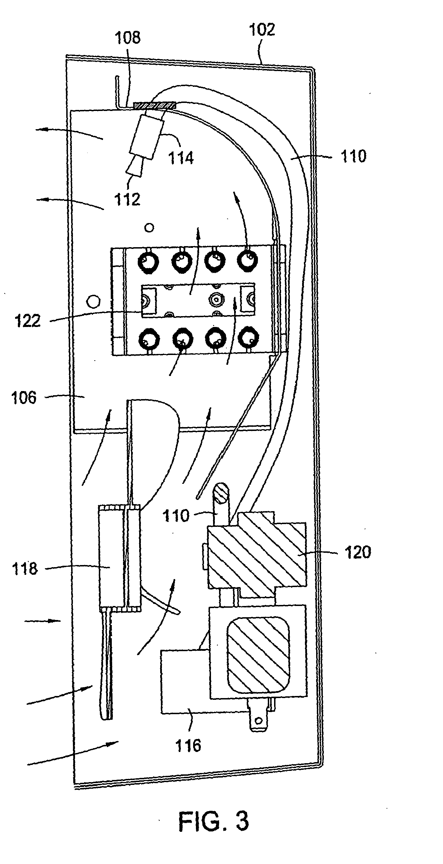

FIG. 3 is a cross-sectional view taken along the 3--3 in FIG. 2. It illustrates the relative location of the various components of the electric unit 100. Furthermore, it illustrates the flow of air through the electric unit 100. First, when the fan 118 is turned on, air is pulled into the electric unit 100 through the slits 154 in the lower part of the front cover 104 (see FIG. 1). The air is directed up through the heating element 122. The warmed air then passes through the electrodes 112, 113 and exit the electric unit 100. The electrodes 112, 113 have a pulse potential between them of approximately 3000 volts and thereby create an electric field, such that water molecules in the air passing through the field break apart into positive hydrogen ions and negative hydroxyl ions. The negative hydroxyl ions then bond to bacteria and viruses and destroying them. It is noted that the bacteria killing capability of the unit 100 can function without the heating element 122. Therefore, a user can set the thermostat 126 to keep the heating element 122 off and still enjoy the benefit of the bacteria killing function.

FIG. 4 is an exploded view of the fan-panel assembly 200 according to an embodiment of the present invention showing various sub-parts. The fan 118 is connected to the motor 120 via a shaft 172. The fan 118 and the motor 120 are mounted to the fan deck 106. The fan 118 is secured through the shaft 172 and held by the fan clip 132. The thermostat 126 also mounted on the fan deck 106.

A thermo sensor 140 is mounted on the element mount 108 and is electrically connected the heating element 122. If the electric unit 100 is heated beyond the pre-determined temperature, the thermo sensor 140 will cut off the circuit to the heating element 122 automatically, and when the electric unit 100 is cooled, the thermo sensor 140 will reconnect the circuit enabling the heating element 122 to function.

The thermo fuse 138 is mounted on the element mount 108 and it is electrically connected to the heating element 122. The thermo fuse 138 is an additional fail-safe mechanism. The thermo fuse 138 is configured to break the circuit to the heating element 122 if the thermo sensor 140 fails. Thus, if the thermo sensor 140 fails to break the circuit if the electric unit 100 overheats, the thermo fuse 138 will break the circuit to the heating element 122. If this happens, the electric unit 100 will need servicing.

As discussed previously, the indicator light 134 is mounted on the fan deck 106. The indicator light 134 is electrically connected to the heating element 122, such that the indicator light 134 illuminates when either the thermo sensor 140 or the thermo fuse 138 activates to cut power to the heating element 122. For example, in the event that the thermo sensor 140 senses that the electric unit 100 is overheated, it will cut off power to the heating element and the indicator light 134 will turn on automatically. When the unit 100 is cooled, the thermo sensor 140 returns power to the heating element and the indicator light 134 will turned off. In the event that the thermo fuse 138 is activated, the indicator light 134 will not turn off until the electric unit 100 is serviced.

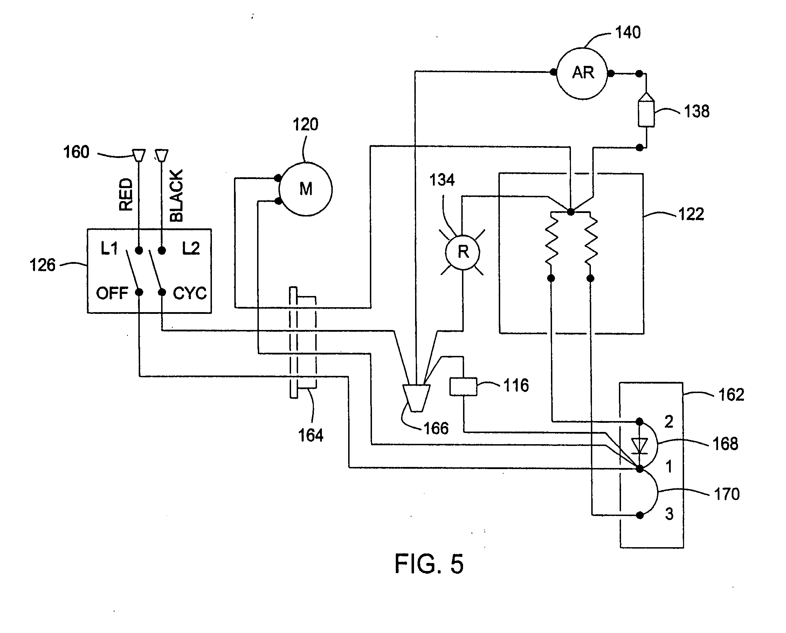

FIG. 5 illustrates a first wiring diagram of an embodiment of the present invention. The wiring diagram illustrates the electrical connectivity of the various elements of the present invention. In the embodiment of the invention shown in FIG. 5, the heating element 122, the motor 120, and the cold plasma generator 116 are in parallel connection.

As shown, the thermostat 126 is electrically connected to the power supply 160. The thermostat 126 is also electrically connected to a wattage selection board 162 through bushing 164. The wattage selection board allows the manufacturer to elect different wattage levels, for example, by selectively removing one or more wires 168,170, the wattage can range between 500 to 2000 watts. The wires 168,170 are electrically connecting to the heating element 122. The change of wattage may affect the maximum heating capacity of the heating element 122. Thus, the manufacturer can manufacture heating units with various maximum heat setting by simply removing one or both electric wires 168, 170.

Furthermore, the thermostat 126 is electrically connected to the thermo sensor 140 through a splice 166. The thermo sensor is electrically connected to the thermo fuse 138. The thermo fuse 138 is further connected to the heating element 122. The motor 120 is electrically connected to the heating element 122 and the wattage selection board 162. The cold plasma generator 116 is electrically connected to the splice 166 and the wattage selection board 162. An indicator light 134 is also electrically connected to the splice 166 and the heating element 122. It is noted that this is a parallel system. When the electric unit is switched on, the heating element 122, the cold plasma generator 116, and the motor 120 will all be powered.

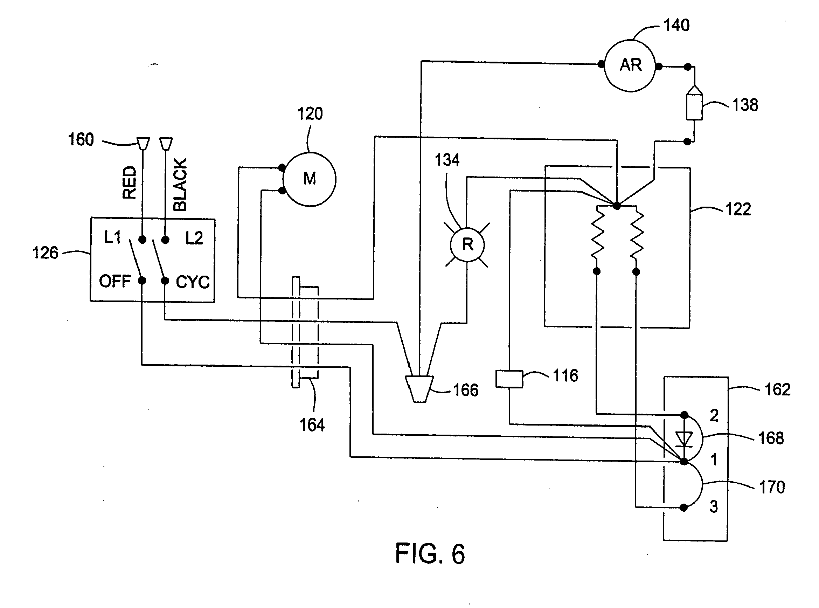

FIG. 6 illustrates a second wiring diagram of another embodiment of the present invention. In this embodiment, the cold plasma generator 116 is electrically connected between the heating element 122 and the wattage selection board 162. The cold plasma generator 116 will be disabled when the thermostat 126 is disconnected from the power source. Alternatively, the cold plasma generator 116 can be integrated with the heating element 122. Thus, the cold plasma generator 116 can be controlled by the thermo sensor 140 and the thermo fuse 138.

In operation, when the electric unit is placed in a room, the heating element 122 generates warm air, the cold plasma generator 116 sends power to the electrodes 112,113. The potential between the electrodes 112,113 creates an electric field. As water molecules in the air pass through the electric field, the water molecules are broken down into hydrogen ions and hydroxyl ions, and the motor 120 drives the fan 118 to blow the heated air and the ions out to the room and draws in cold air. The hydroxyl ions will bond with bacteria and viruses of the room. After several cycles, the room warms up and many of the bacteria and viruses will be rendered inert.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. An electrical unit, comprising:
an air circulator configured to generate an air stream;
a cold plasma generator operably connected to a first and a second high voltage wires, the cold plasma generator configured to produce a pulse voltage differential between the first and second high voltage wires;
a first electrode electrically connected to the first high voltage wire; and
a second electrode electrically connected to the second high voltage wire; the first and second electrodes configured to generate a high pulse voltage electric field in the air stream.

2. The electrical unit of claim 1, wherein the first and the second electrodes are each covered at least in part by plastic brackets.

3. The electrical unit of claim 1, wherein the first and the second electrodes are between about 1.20 inches to about 1.30 inches apart.

4. The electrical unit of claim 3, wherein the first electrode and the second electrode are about 1.26 inches apart.

5. The electrical unit of claim 1, wherein the cold plasma generator generates between about 2500 to about 6000 volts.

6. The electrical unit of claim 5, wherein the cold plasma generator generates an electric pulse of about 3000 volts between the first and the second electrodes.

7. The electrical unit of claim 6, wherein the electric pulse has a cycle of between about 3 to about 4 milliseconds.

8. The electrical unit of claim 1, wherein the first and the second electrodes are carbon brushes.

9. The electrical unit of claim 1, further comprising:
a controller;
a heating element electrically connected to and controlled by the controller;
a motor electrically connected to the air circulator;
a thermo sensor electrically connected to the heating element, and configured to send a signal to the electrical unit to turn off the heating element when a temperature associated with the electrical unit raises above a predetermined temperature;
a thermal fuse electrically connected between the heating element and the thermo sensor;
an indicator light electrically connected to the heating element, such that the indicator light illuminates when at least one of the thermo sensor and the thermo fuse fail; and
a wattage selection board electrically connected to the controller and the heating element, the wattage selection board is configured to modify a wattage output of the electrical unit.

10. The electrical unit of claim 1, further comprising:
a back cover, wherein the cold plasma generator is mounted on the back cover;
a front cover fitted over the back cover, the front cover having at least one slit to allow air flow through the at least one slit;
an fan deck mounted between the back cover and the front cover, wherein the fan deck is connected to the motor and the air circulating unit; and
an element mount, having a gradually curved rear section, fitted on the fan panel assemble, such that the heating element is situated between the element mount and the fan panel assemble.

11. The electrical unit of claim 10, wherein the first and the second electrodes are mounted on the element mount.

12. The electrical unit of claim 10, wherein the first and the second electrodes are located above the heating element, and the heating element is located above the air circulator, such that air enters the electrical unit though the air circulator, passes over the heating element, and passes though an electric field generated by the first and the second electrodes before exiting the electrical unit.

13. The electrical unit of claim 1, wherein the cold plasma generator generates an electric field pulse that breaks apart water molecules in the air stream into positive hydrogen ions and negative hydroxyl ions.

14. An electrical apparatus comprising:
means for circulating an air stream;
means for generating high voltage connected to the means for circulating, means for generating high voltage operably connected to a first means and a second means for transmitting high voltage, the means for generating high voltage configured to produce a pulse voltage between the first means and the second means for transmitting high voltage;
a first means for generating a high voltage electric field in the air stream electrically connected to the first means for transmitting high voltage; and
a second means for generating a high voltage electric field in the air stream electrically connected to the second means for transmitting high voltage.

15. The electrical apparatus of claim 14, wherein the first means and the second means for generating the electric field are between about 1.20 inches to about 1.30 inches apart.

16. The electrical apparatus of claim 15, wherein the first means and the second means for generating the electric field are about 1.26 inches apart.

17. The electrical apparatus of claim 14, wherein the means for generating high voltage generates about between 2500 to 6000 volts of power.

18. The electrical apparatus of claim 17, wherein the means for generating high voltage generates an electric pulse of about 3000 volts between the first means and the second means for generating the electric field.

19. The electrical apparatus of claim 18, wherein the electric pulse has a cycle of between about 3 to about 4 milliseconds.

20. The electrical apparatus of claim 14, further comprising:
means for controlling a temperature;
means for heating electrically connected to the means for controlling the temperature;
a motor electrically connected to the means for circulating the air stream;
means for sensing temperature electrically connected to the means for heating, and sends a signal to the electrical apparatus to turn off the heating element when the temperature raises above a pre-determined temperature;
a thermal fuse electrically connected between the means for heating and the means for sensing; and
means for indicating electrically connected to the means for heating, such that the means for indicating illuminates when at least one of the means for sensing and the thermo fuse fail.

21. A method of making an electrical apparatus, comprising:
placing a cold plasma generator inside a housing;
connecting a first and a second high voltage wires to the cold plasma generator;
connecting a first electrode to the first high voltage wire;
connecting a second electrode to the second high voltage wire; and
placing an air circulator inside the housing, such that the air circulator moves air through an electric field generated by the first and second electrodes.

22. The method of making an electrical apparatus of claim 21, comprising:
connecting a motor to the air circulator;
placing a heating element inside the housing;
connecting a thermo sensor to the heating element;
connecting a thermal fuse between the heating element and the thermo sensor; and
placing an indicator light in the housing.
